# EUROPEAN PATENT APPLICATION

(11) **EP 4 837 599 A1**
(43) Date of publication of application: **11.11.2026**
(21) Application number: 24914658.0
(22) Date of filing: 05.01.2024
(51) Int. Cl.: A61M 5/00, A61M 5/20, A61B 5/15

(54) **BODY FLUID SAMPLING APPARATUS AND METHOD**

(71) Applicant: MGI Tech Co., Ltd., Shenzhen, Guangdong 518083 (CN)
(72) Inventor: GU, Xianyong, Shenzhen, Guangdong 518083 (CN); YANG, Meng, Shenzhen, Guangdong 518083 (CN); YAO, Fei, Shenzhen, Guangdong 518083 (CN); LIU, Qinglin, Shenzhen, Guangdong 518083 (CN)
(74) Representative: Metida
(86) International application number: PCT/CN2024/070856
(87) International publication number: WO 2025/145429

(57) **Abstract**

A body fluid sampling apparatus and method are provided. The apparatus includes a puncture module, a driving module, a resetting module, and a first locking module. The puncture module includes a puncture member. The driving module includes a first actuator with driving potential energy. The first actuator drives the puncture member to protrude from a sampling port of the housing along a first direction from the first position and move to the second position. The resetting module includes a second actuator with resetting potential energy. The second actuator drives the puncture member at the second position to retract into the sampling port along an opposite direction of the first direction. The first locking module can lock the resetting module, and can also unlock the resetting module when the puncture member reaches the second position, allowing the second actuator to retract the puncture member. The entire process, from the puncture member reaching the second position, to the first locking module unlocks the resetting module, and then to the second actuator returning the puncture member, is interlinked, which can reduce the duration of the puncture member remaining within the barrier.

## Description

### FIELD

The subject matter relates to the field of medical devices, and more particularly, to a body fluid sampling apparatus and a body fluid sampling method.

### BACKGROUND

At present, the sampling of human body fluid (such as blood) mainly relies on the assistance of professional medical personnel. With the development of point-of-care testing (POCT) and changes in testing models of future hospitals, the existing blood sampling methods will not be able to meet the people's needs for self-service sampling.

Therefore, a simple and easy-to-use blood sampling apparatus is needed, which enables nonprofessional personnel to independently collect the blood without any professional skills. There is an apparatus in related arts that employs a pressing structure for self-service blood collection. The principle of such apparatus is that an internal needle may be quickly inserted into the skin when the pressing structure is pressed by a user, and at this time, an elastic member at the bottom of the needle may be elastically compressed. After the user releases the pressing structure, the elastic member at the bottom rebounds and pushes the needle back to its original position, thereby completing the blood sampling. However, before the user releases the pressing structure, the needle will remain in the skin for a long time, which may cause discomfort and pain to the human body, thereby reducing the user experience.

### SUMMARY

To overcome the above shortcomings, a body fluid sampling apparatus is needed, which is simple and easy-to-use and may reduce the time duration of the needle remaining in the skin.

Additionally, a body fluid sampling method applied to the body fluid sampling apparatus mentioned above is also needed.

A first aspect of the present application provides a body fluid sampling apparatus, including a bottom surface configured to be in contact with a barrier. The body fluid sampling apparatus further includes a puncture module, a driving module, a resetting module, and a first locking module. The puncture module includes a puncture member. The driving module includes a first actuator with a driving potential energy, the first actuator is configured to drive the puncture member from a first position to protrude from a sampling port of the bottom surface along a first direction and move to a second position. The resetting module includes a second actuator with a resetting potential energy, the second actuator is configured to drive the puncture member to retract from the second position into the sampling port along an opposite direction of the first direction. The first locking module is configured to lock the resetting module, the first locking module is associated with the second position to unlock the resetting module in response to that the puncture member is at the second position, thereby causing the second actuator to release the resetting potential energy which drives the puncture member to retract. A time duration from the puncture member protruding from the sampling port to the puncture member retracting into the sampling port is defined as T, and T is less than or equal to 5 milliseconds.

A second aspect of the present application provides a body fluid sampling apparatus, including a bottom surface configured to be in contact with a barrier. The body fluid sampling apparatus further includes a puncture module, a driving module, a resetting module, and a first locking module. The puncture module includes a puncture member. The driving module includes a first actuator with a driving potential energy, the first actuator is configured to drive the puncture member from a first position to protrude from a sampling port of the bottom surface along a first direction and move to a second position. The resetting module includes a second actuator with a resetting potential energy, the second actuator is configured to drive the puncture member to retract from the second position into the sampling port along an opposite direction of the first direction. The first locking module is configured to lock the resetting module, the first locking module is associated with the second position to unlock the resetting module in response to that the puncture member is at the second position, thereby causing the second actuator to release the resetting potential energy which drives the puncture member to retract. A time duration from the puncture member reaching the second position to the puncture member starting to move along the opposite direction under drive of the second actuator is defined as t, and t is less than or equal to 0.5 milliseconds.

A third aspect of the present application provides a body fluid sampling apparatus, including a bottom surface configured to be in contact with a barrier. The body fluid sampling apparatus further includes a puncture module, a driving module, a resetting module, and a first locking module. The puncture module includes a puncture member. The driving module includes a first actuator with a driving potential energy, the first actuator is configured to drive the puncture member from a first position to protrude from a sampling port of the bottom surface along a first direction and move to a second position. The resetting module includes a second actuator with a resetting potential energy, the second actuator is configured to drive the puncture member to retract from the second position into the sampling port along an opposite direction of the first direction. The first locking module is configured to lock the resetting module, the first locking module is associated with the second position to unlock the resetting module in response to that the puncture member is at the second position, thereby causing the second actuator to release the resetting potential energy which drives the puncture member to retract. A time duration from the puncture member protruding from the sampling port to the puncture member retracting into the sampling port is defined as T, a time duration from the puncture member reaching the second position to the puncture member starting to move along the opposite direction under drive of the second actuator is defined as t, and t/T is less than or equal to 10 %.

A fourth aspect of the present application provides a body fluid sampling method, including attaching a bottom surface of a body fluid sampling apparatus to a surface of a barrier, wherein the body fluid sampling apparatus includes a puncture module, a driving module, a resetting module, and a first locking module, the puncture module includes a puncture member, the driving module includes a first actuator with a driving potential energy, the resetting module includes a second actuator with a resetting potential energy, and the first locking module is configured to lock the resetting module. The puncture member is driven by the first actuator from a first position to protrude from a sampling port of the bottom surface, puncture the barrier, and move to a second position along a first direction. The resetting module is unlocked by the first locking module. The puncture member is driven by the second actuator to retract from the second position to exit the barrier and into the sampling port along an opposite direction of the first direction. A time duration from the puncture member protruding from the sampling port to the puncture member retracting to the sampling port is defined as T, a time duration from the puncture member reaching the second position to the puncture member starting to move along the opposite direction under drive of the second actuator is defined as t, and t/T is less than or equal to 10 %.

A fifth aspect of the present application provides a body fluid sampling method, including driving a driving shaft of a body fluid sampling apparatus to move, thereby causing the driving shaft to move a puncture member to protrude from a sampling port along a puncture direction to puncture a barrier, wherein the body fluid sampling apparatus comprises a bottom surface in contact with the barrier, and the sampling port is defined at the bottom surface. The puncture member is moved by the driving shaft to a second position to trigger a resetting module, thereby causing the resetting module to release a pre-stored resetting potential energy which drives the driving shaft to move the puncture member away from the barrier and retract into the sampling port along a resetting direction opposite to the puncture direction. A time duration from the puncture member protruding from the sampling port to the puncture member retracting to the sampling port is defined as T, a time duration from the puncture member reaching the second position to the puncture member starting to move along the opposite direction under drive of the resetting module is defined as t, and t/T is less than or equal to 10 %.

The present application has a simple structure and is easy to operate. The sampling and collection of body fluid may be completed by attaching the body fluid sampling apparatus to the surface of the barrier without the need for professional medical assistance. During the puncture process, the first actuator releases the driving potential energy that drives the puncture member of the puncture module to move along the first direction. When the puncture member moves along the first direction to the second position, the first locking module will be triggered to unlock the resetting module, thereby causing the second actuator to release the resetting potential energy that drives the puncture member back along the opposite direction of the first direction. The entire process from the puncture member reaching the second position, to the first locking module unlocking the resetting module, and then to the second actuator driving the puncture member back, is interlinked. The above process does not require the user to press the puncture member to cause the puncture member to protrude out, thus there is no need for the user to remove the external force to retract the puncture member. The above linkage process may reduce the time duration of the puncture member remaining within the barrier, thereby reducing the discomfort and pain caused by puncture and improving the user experience.

### BRIEF DESCRIPTION OF THE DRAWINGS

FIG. 1 is a schematic diagram of a body fluid sampling apparatus according to an embodiment of the present application.
FIG. 2 is a cross-sectional view taken along II-II of the body fluid sampling apparatus shown in FIG. 1.
FIG. 3 is a cross-sectional view of the body fluid sampling apparatus shown in FIG. 2, wherein a puncture member reaches a second position along a first direction.
FIG. 4 is a cross-sectional view of the body fluid sampling apparatus shown in FIG. 3, wherein a first locking module is unlocked.
FIG. 5 is a cross-sectional view of the body fluid sampling apparatus shown in FIG. 4, wherein the puncture member retracts along an opposite direction of the first direction.
FIG. 6 is a diagram showing speeds of the puncture member of the body fluid sampling apparatus shown in FIG. 2 with respect to times.
FIG. 7 is a flowchart of a body fluid sampling method provided in an embodiment of the present application.
FIG. 8 is a flowchart of a body fluid sampling method according to an embodiment of the present application.
FIG. 9 is a photo captured by a high-speed camera according to an embodiment of the present application, which shows an entire movement process of the puncture member of the body fluid sampling apparatus.

**Description of numerals of main components:**

| | |
|---|---|
| Puncture module | 10 |
| Puncture member | 11 |
| Fixing base | 12 |
| Driving module | 20 |
| First actuator | 21 |
| Driving shaft | 22 |
| Resetting module | 30 |
| Second actuator | 31 |
| Shaft base | 32 |
| First locking module | 40 |
| Moving block | 41 |
| Third actuator | 42 |
| Second locking module | 50 |
| Bracket | 60 |
| Receiving space | 61 |
| Housing | 70 |
| Bottom surface | 71 |
| Body fluid sampling apparatus | 100 |
| First elastic member | 210 |
| First flange | 220 |
| Pushing portion | 320 |
| Second elastic member | 310 |
| Second flange | 321 |
| Slot | 410 |
| Step portion | 621 |
| Sampling port | 710 |
| Engagement depth | H |
| Range of motion | L |
| First position | P1 |
| Second position | P2 |
| First direction | X |
| Second direction | Y |
| Third direction | Z |

### DETAILED DESCRIPTION

Implementations of the disclosure will now be described, by way of embodiments only, with reference to the drawings. The described embodiments are only some embodiments of the present disclosure, rather than all the embodiments.

It should be noted that when a component is referred to as being "arranged on" another component, the component can be directly on another component or a middle component may exist therebetween. When a component is considered to be "mounted to" another component, the component can be directly on another component or a middle component may exist therebetween. The term "and/or" as used herein means any combinations of one or more related listed items.

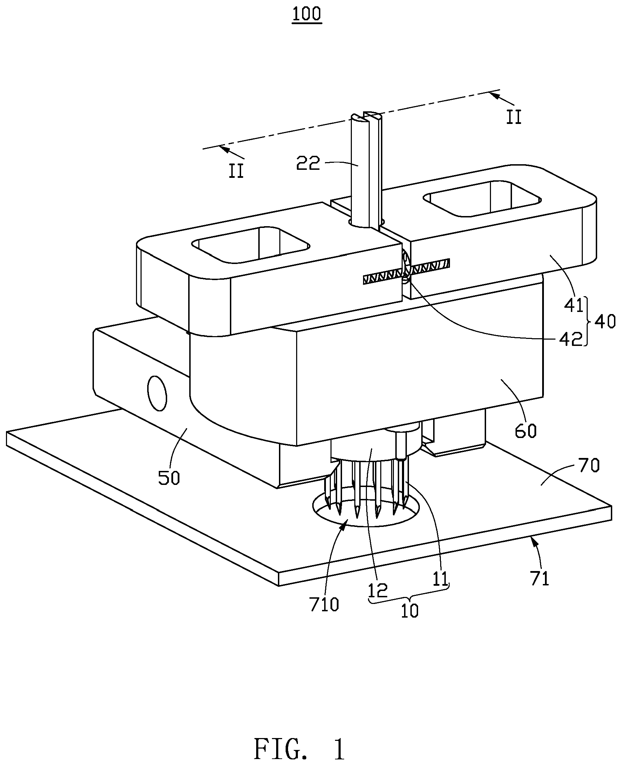

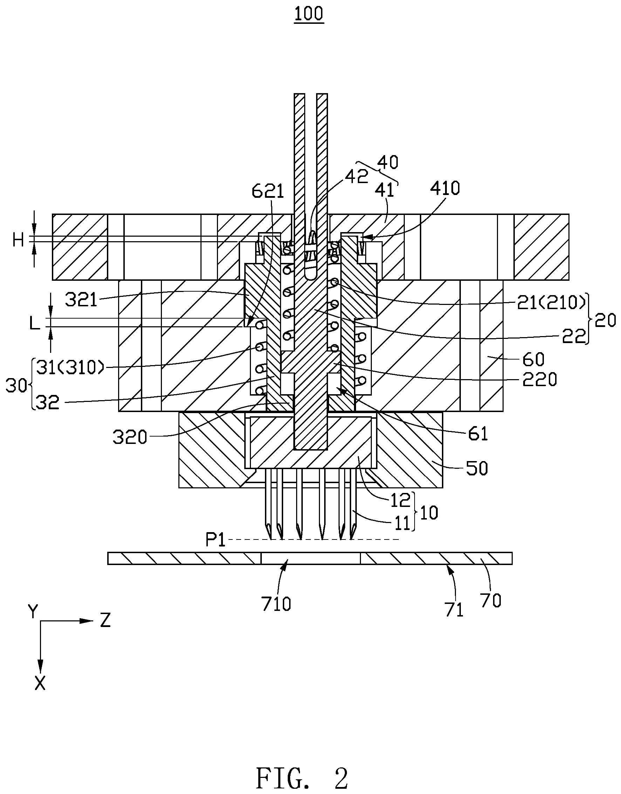

Referring to FIGS. 1 and 2, a body fluid sampling apparatus 100 is provided according to an embodiment of the present application, which may puncture a barrier (such as human skin) to collect a body fluid, such as blood sample or interstitial fluid sample, from the human body. The body fluid sampling apparatus 100 includes a housing 70, a puncture module 10, a driving module 20, a resetting module 30, and a first locking module 40. Each of the puncture module 10, the driving module 20, the resetting module 30, and the first locking module 40 is located in the housing 70. The housing 70 has a bottom surface 71 for coming in contact with the barrier. The bottom surface 71 defines a sampling port 710. For simplicity, only the bottom surface 71 of the housing 70 is shown in FIGS. 1 and 2, and the remaining portions of the housing 70 are not shown.

The puncture module 10 includes at least one puncture member 11. The figures show that there are a number of puncture members 11. The puncture member 11 is used to protrude from the sampling port 710 along a first direction X (i.e. a puncture direction) under a driving force, so as to puncture the barrier. In some embodiments, the puncture module 10 may further include a fixing base 12 for fixing the puncture members 11. When the above driving force is applied onto the fixing base 12, the fixing base 12 drives the puncture member 11 to move along the first direction X and then protrude from the sampling port 710. The puncture member 11 may be a solid needle made of a metal material. For example, the puncture member 11 may be a microneedle. It can be understood that there may be a flow channel (not shown) inside the housing 70, and after the barrier is punctured, the body fluid may flow out towards the flow channel. Such a technology is known in the arts and will not be repeated here.

Furthermore, the body fluid sampling apparatus 100 may further include a second locking module 50 for locking the puncture module 10. For example, when the body fluid sampling apparatus 100 is not in use, the second locking module 50 may fix the fixing base 12, thereby avoiding any unnecessary damage to the human body caused by the puncture member 11 protruding from the sampling port 710 under an unintended operation of the body fluid sampling apparatus 100. When the body fluid sampling apparatus 100 is used for body fluid sampling, the second locking module 50 may unlock the fixing base 12, such that the fixing base 12 may drive the puncture member 11 to move along the first direction X and then protrude from the sampling port 710. In some embodiments, the second locking module 50 may be a sliding block that may slide within the housing 70 along a second direction Y perpendicular to the first direction X. The sliding block may support the surface of the fixing base 12 having the puncture member 11 along the first direction X, thereby locking the fixing base 12 along the first direction X. When the sliding block slides along the second direction Y and is separated from the fixing base 12, the sliding block unlocks the fixing base 12. The sliding block may slide under an external force to unlock the fixing base 12, but the present application is not limited thereto. For example, the user may operate a relevant button provided on the housing 70 to control the sliding block to slide.

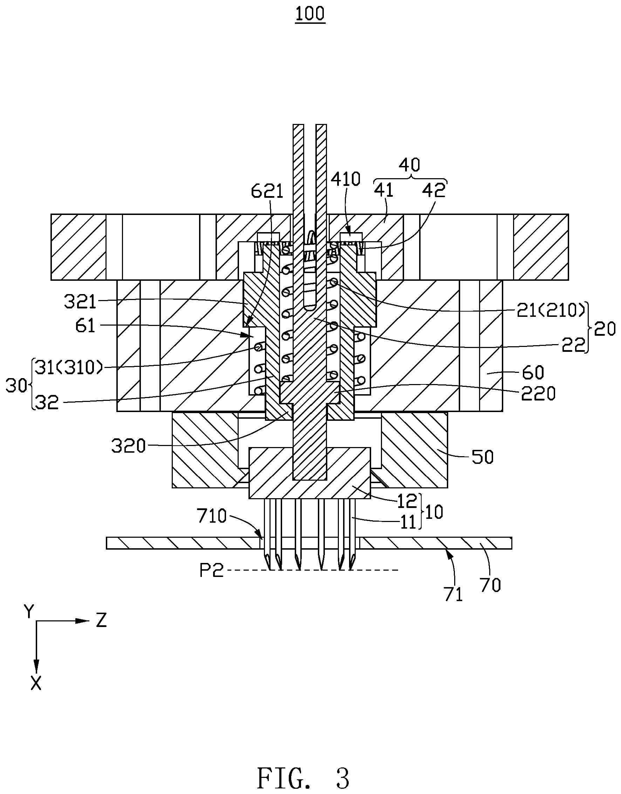

The driving module 20 is fixed to the puncture module 10. The driving module 20 includes a first actuator 21. When the body fluid sampling apparatus 100 is not in use, the first actuator 21 has a driving potential energy. The first actuator 21 may release the driving potential energy that provides the driving force mentioned above, which drives the puncture member 11 from the first position P1 to move through the sampling port 710, puncture the barrier, and then reach the second position P2 along the first direction X. As shown in FIG. 2, the first position P1 may be understood as an initial position of the puncture member 11, that is, a position of the puncture member 11 when the body fluid sampling apparatus 100 is not in use. The puncture member 11, which is at the initial position, is completely received within the housing 70, thereby avoiding any unnecessary damage caused by an accidental touch of the user. As shown in FIG. 3, the second position P2 may be understood as an extreme position that the puncture member 11 can reach along the first direction X after puncturing the barrier, which corresponds to a maximum depth that the puncture member 11 can puncture. The first position P1 and the second position P2 labeled in FIGS. 2 and 3 are the positions of the tip of the puncture member 11. It can be understood that the above labeling manner is only an example, and the first position P1 and the second position P2 may also be positions of other portions of the puncture member 11. FIG. 2 illustrates the first position P1 as an example. It can be understood that the first position P1 only needs to be located on a side of the bottom surface 71 such that the tip of the puncture member 11 does not protrude from the housing 70 when the body fluid sampling apparatus 100 is not in use. For example, the first position P1 may also be located inside the sampling port 710.

Furthermore, the driving module 20 may further include a driving shaft 22 fixed to the puncture module 10. For example, the driving shaft 22 may be fixed to the fixing base 12. The first actuator 21 includes a first elastic member 210 surrounding the driving shaft 22. The outer circumference of the driving shaft 22 is provided with a first flange 220, and the first elastic member is located on a side of the first flange 220 away from the fixing base 12. One end of the first elastic member 210 is elastically resisted against the first flange 220. Therefore, the first elastic member 210 may push the first flange 220 to move along the first direction X, thereby moving the puncture member 11 from the first position P1 along the first direction X. In some embodiments, the first elastic member 210 is a helical cylindrical spring that is pre-compressed. The first elastic member 210 may also be other types of elastic structures suitable to be compressed and provide the driving force to the puncture member 11.

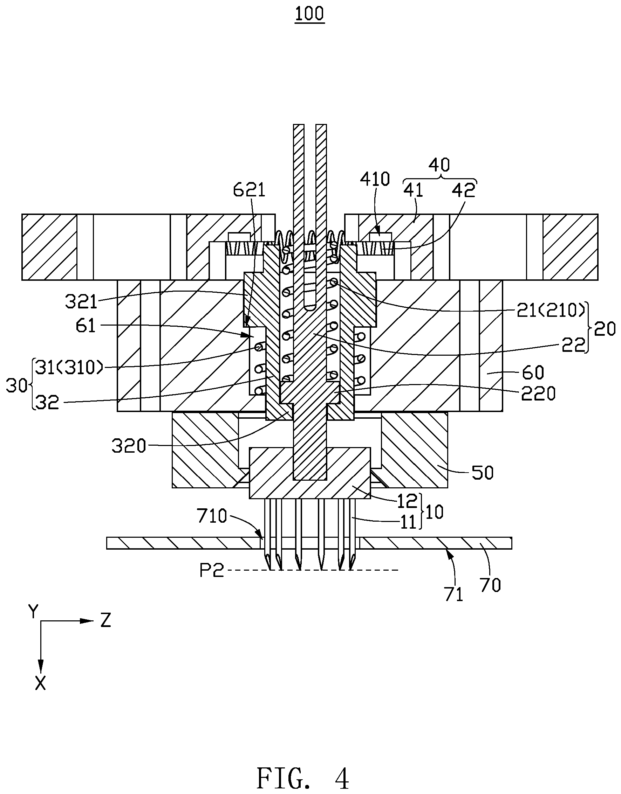

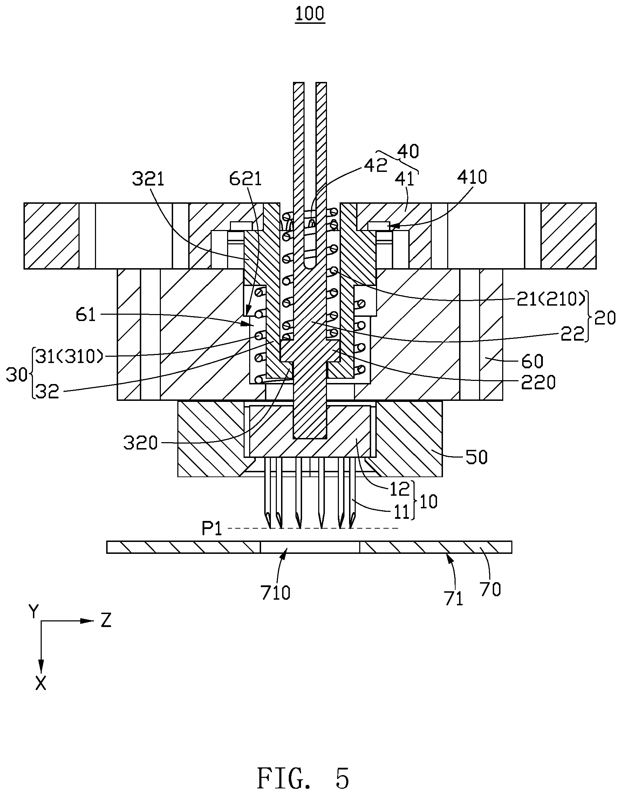

The resetting module 30 includes a second actuator 31. When the body fluid sampling apparatus 100 is not in use, the second actuator 31 has a resetting potential energy. As shown in FIGS. 4 and 5, the second actuator 31 may release the resetting potential energy that moves the puncture member 11 from the second position P2 along the opposite direction of the first direction X (i.e., the resetting direction) until the puncture member 11 exits the barrier, and the puncture member 11 further retracts into the housing 70 through the sampling port 710, thereby completing the sampling. The puncture member 11 may return to the first position P1 or a position deviated from the first position P1, which is not limited in the present application.

Furthermore, the resetting module 30 may further include a shaft base 32 that is linked to the driving shaft 22. The second actuator 31 includes a second elastic member 310 surrounding the shaft base 32. In some embodiments, the bottom portion of the shaft base 32 near the fixing base 12 may extend inward to form a pushing portion 320, and the first flange 220 may be resisted against the pushing portion 320, thereby linking the shaft base 32 with the driving shaft 22. As shown in FIG. 2, when the body fluid sampling apparatus 100 is not in use, there is a certain distance between the first flange 220 and the pushing portion 320 along the first direction X. As shown in FIG. 3, when the puncture module 10 is unlocked, the first elastic member 210 releases the compression potential energy that pushes the first flange 220 of the driving shaft 22 to move along the first direction X, thereby moving the puncture member 11 from the first position P1 along the first direction X. During the above process, the first flange 220 will first come into contact with the pushing portion 320, and then move the shaft base 32 along the first direction X through the pushing portion 320. Therefore, the shaft base 32 is linked with the driving shaft 22 along the first direction X. When the second elastic member 310 releases the compression potential energy that drives the shaft 22 base to retract along the opposite direction of the first direction X, the pushing portion 320 of the shaft base 32 moves the first flange 220 along the opposite direction of the first direction X, thereby moving the puncture member 11 along the opposite direction of the first direction X through the driving shaft 22. Therefore, the shaft base 32 is linked with the driving shaft 22 along the opposite direction of the first direction X. In some embodiments, the second elastic member 310 is a helical cylindrical spring that is pre-compressed. The second elastic member 310 may also be other types of elastic structures suitable to be compressed and provide the driving force to the puncture member 11.

Specifically, the outer circumference of the shaft base 32 may be provided with a second flange 321, and the second elastic member 310 is located on a side of the second flange 321 near the fixing base 12. One end of the second elastic member 310 is elastically resisted against the second flange 321. Therefore, the second elastic member 310 may act on the second flange 321, thereby pushing the shaft base 32 back along the opposite direction of the first direction X through the second flange 321. During the process of the first flange 220 moving the shaft base 32 along the first direction X through the pushing portion 320, the second elastic member 310 may be further compressed.

The first locking module 40 may lock the resetting module 30. Specifically, when the body fluid sampling apparatus 100 is not in use as shown in FIG. 2, the first locking module 40 may lock the shaft base 32 along the first direction X. When the puncture member 11 starts to move from the first position P1 along the first direction X, the first locking module 40 still locks the shaft base 32 along the first direction X, such that the second elastic member 310 cannot push the shaft base 32 along the opposite direction of the first direction X through the second flange 321. As shown in FIGS. 3 to 5, the first locking module 40 is associated to the second position P2. In the present application, the term "associate" indicates that there is an interrelated relationship between the first locking module 40 and the second position P2. Therefore, when the puncture member 11 reaches the second position P2, the first locking module 40 unlocks the shaft base 32, such that the second actuator 31 may release the resetting potential energy that drives the puncture member 11 to exit the barrier along the opposite direction of the first direction X and retract into the housing 70 through the sampling port 710.

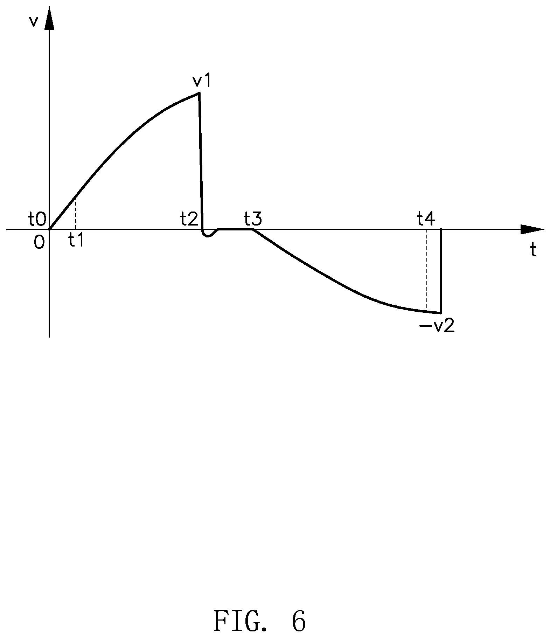

FIG. 6 shows a relationship between speeds of the puncture member 11 and times, when the puncture member 11 does not puncture the barrier. First, the puncture member 11 will start from the initial position along the first direction X, pass through the sampling port 710, and then reach the second position P2. During the process from the initial position to the second position P2, a speed v1 of the puncture member 11 increases. When the second position P2 is reached, the speed v1 of the puncture member 11 decreases to zero in a very short time duration, and the puncture member 11 will experience a small rebounding process near the second position P2. Within a short time duration after the rebounding process, the puncture member 11 starts to move along the opposite direction of the first direction X and the speed v2 increases, and finally the puncture member 11 retracts into the housing 70 through the sampling port 710. It should be understood that the speeds v1 and v2 in this embodiment are only for illustrative purposes, and are not intended to limit the values of the speeds of the puncture member 11 moving along different directions. The time point at which the puncture member 11 starts to move along the first direction X is defined as t0 (t0 may coincide with the origin of the coordinate axis). The time point at which the puncture member 11 exits the sampling port 710 is defined as t1. The time point at which the puncture member 11 reaches the second position P2 is defined as t2. The time point at which the puncture member 11 starts to retract along the opposite direction of the first direction X under the drive of the second actuator 31 is defined as t3. The time point at which the puncture member 11 retracts into the housing 70 through the sampling port 710 is defined as t4.

In the present application, the time duration from the puncture member 11 protruding from the sampling port 710 to the puncture member 11 retracting into the housing 70 is defined as T (i.e., the time duration from t1 to t4), wherein T is less than or equal to 5 milliseconds. Therefore, the time duration required from the puncture member 11 protruding from the sampling port 710 to the puncture member 11 retracting into the housing 70 is very short. Therefore, when the body fluid sampling apparatus 100 is used to puncture the barrier for body fluid sampling, the time duration of the puncture member 11 remaining inside the barrier may be reduced. It can be understood that when the elastic coefficient of the first elastic member 210 or the second elastic member 310 changes, the weight of the relevant modules in the apparatus may be designed accordingly to compensate for the influence of the elastic coefficient, thereby ensuring that the above time duration T will not change due to unilateral adjustment of the elastic coefficient.

In some embodiments, the time duration from the puncture member 11 reaching the second position P2 to the puncture member 11 starting to move along the opposite direction under the drive of the second actuator 31 is defined as t (i.e., the time duration from t2 to t3), wherein t is less than or equal to 0.5 milliseconds. Therefore, the time duration from the puncture member 11 reaching the second position P2 to the puncture member 11 starting to move along the opposite direction is very short. Therefore, when the body fluid sampling apparatus 100 is used to puncture the barrier for body fluid sampling, the time duration of the puncture member 11 remaining inside the barrier may be reduced. In the present application, the expression "starting to move along the opposite direction" means starting to move regularly and continuously along the opposite direction under the drive of the second actuator 31. The above expression excludes the condition that the puncture member 11 slightly rebounds near the second position P2.

In some embodiments, the time duration from the puncture member 11 reaching the second position P2 to the puncture member 11 starting to move along the opposite direction under the drive of the second actuator 31 is defined as t (i.e., the time duration from t2 to t3), where t/T is less than or equal to 10 %. When the body fluid sampling apparatus 100 is used to puncture the barrier, the ratio of the time duration from the puncture member 11 reaching the second position P2 to the puncture member 11 starting to move along the opposite direction under the drive of the second actuator 31, with respect to the time duration from the puncture member 11 protruding from the sampling port 710 to the puncture member 11 retracting into the housing 70, is substantially less than or equal to 10 %. Therefore, the time duration from the puncture member 11 reaching the second position P2 to the puncture member 11 starting to move along the opposite direction under the drive of the second actuator 31 accounts for a very small ratio of the total time duration, which may also reduce the time duration of the puncture member 11 remaining inside the barrier.

In the present application, when the puncture module 10 is unlocked, the first actuator 21 releases the driving potential energy that drives the puncture member 11 of the puncture module 10 to move along the first direction X. When the puncture member 11 moves along the first direction X to the designated position, that is, the second position P2, the first locking module 40 will be triggered to unlock the resetting module 30, thereby causing the second actuator 31 to release the resetting potential energy that drives the puncture member 11 back into the housing 70 along the opposite direction of the first direction X. The entire process from the puncture member 11 reaching the second position P2, to the first locking module 40 unlocking the resetting module 30, and then to the second actuator 31 driving the puncture member 11 back, is interlinked. The above process does not require the user to press the puncture member 11 to cause the puncture member 11 to protrude out, thus there is no need for the user to remove the external force to retract the puncture member 11. The above linkage process may reduce the time duration of the puncture member 11 remaining within the barrier, thereby reducing the discomfort and pain caused by puncture and improving the user experience.

As shown in FIGS. 2 and 3, in some embodiments, one end of the first elastic member 210 away from the first flange 220 is elastically resisted against the first locking module 40. That is, the first elastic member 210 may be elastically held between the first locking module 40 and the first flange 220. Considering the fact that each of the first elastic member 210 and the second elastic member 310 are in a compressed state, the compression potential energy of the first elastic member 210 will provide a pushing force onto the first flange 220 along the first direction X, which further pushes the pushing portion 320 of the shaft base 32 along the first direction X through the first flange 220. At the same time, the compression potential energy of the second elastic member 310 will provide a reverse pushing force onto the second flange 321 of the shaft base 32, which further pushes the first flange 220 through the pushing portion 320. Therefore, the elastic coefficient of the first elastic member 210 is designed to be greater than that of the second elastic member 310. Therefore, after the second locking module 50 unlocks the puncture module 10, the compression potential energy of the first elastic member 210 is sufficient to counteract the compression potential energy of the second elastic member 310, thereby allowing the puncture member 11 to smoothly move along the first direction X to the second position P2.

Furthermore, as shown in FIG. 4, the first locking module 40 is movable arranged relative to the first elastic member 210, such that the first locking module 40 is separated from the first elastic member 210 when the puncture member 11 reaches the second position P2. Therefore, during the process of the second elastic member 310 driving the puncture member 11 back from the second position P2 along the opposite direction of the first direction X, the end of the first elastic member 210 away from the first flange 220 is no longer elastically resisted against the first locking module 40. That is, the first locking module 40 releases the constraint on the end of the first elastic member 210. Therefore, during the process of the second elastic member 310 driving the puncture member 11 back, the movement of the shaft base 32 and the driving shaft 22 is no longer limited by the compression potential energy of the first elastic member 210, thereby avoiding the situation where the first elastic member 210 with a larger elastic coefficient acts on the driving shaft 22 and the shaft base 32 to prevent the puncture member 11 from moving back.

As shown in FIG. 2, in some embodiments, the body fluid sampling apparatus 100 further includes a bracket 60. The bracket 60 is fixed within the body fluid sampling apparatus 100. For example, the bracket 60 may be fixed inside the housing 70 by fasteners such as screws. The bracket 60 supports the driving module 20 and the resetting module 30. Along the first direction X, bracket 60 is located between the first locking module 40 and the second locking module 50. The shaft base 32 is movable arranged within the bracket 60 along the first direction X. The shaft base 32 has a predetermined range of motion along the first direction X within the bracket 60, such that the puncture member 11 is stopped at the second position P2. Specifically, the bracket 60 defines a receiving space 61, and at least a portion of the driving module 20 and at least a portion of the resetting module 30 are received in the receiving space 61. The inner sidewall of the receiving space 61 is provided with a step portion 621. When the body fluid sampling apparatus 100 is not in use, there is a gap between the step portion 621 and the second flange 321 along the first direction X, and the gap is the range of motion of the shaft base 32 within the bracket 60 along the first direction X. As shown in FIG. 3, when the first elastic member 210 pushes the first flange 220 of the driving shaft 22 to move along the first direction X, the first flange 220 may first come into contact with the pushing portion 320, and then drive the shaft base 32 to move along the first direction X through the pushing portion 320. When the shaft base 32 moves to cause the step portion 621 to be in contact with the second flange 321, the driving shaft 22 stops moving, thereby stopping the puncture member 11 at the second position P2. As shown in FIG. 6, due to the rigid stopping function of the step portion 621, the velocity v1 of the puncture member 11 may decrease to zero in a very short time duration when the puncture member 11 reaches the second position P2.

As shown in FIG. 2, furthermore, the first locking module 40 may define a slot 410, and a portion of the shaft base 32 (e.g., one end of the shaft base 32 away from the pushing portion 320) is engaged with the slot 410, thereby locking the first locking module 40 to the resetting module 30. In some embodiments, the first locking module 40 includes two moving blocks 41 arranged along the third direction Z and a third actuator 42 elastically resisted against between the two moving blocks 41. Each moving block 41 defines with one slot 410. Therefore, when a portion of the shaft base 32 is engaged with the slot 410, the two moving blocks 41 is fixed relative to each other. As shown in FIGS. 3 and 4, when the end of the shaft base 32 is separated from the slot 410, the third actuator 42 pushes the two moving blocks 41 away from each other along the third direction Z. At this time, the first locking module 40 unlocks the resetting module 30, such that the second actuator 31 may drive the puncture member 11 to exit the barrier along the opposite direction of the first direction X. Since a certain time duration may be needed for the two moving blocks 41 to move away from each other to unlock the resetting module 30, as shown in FIG. 6, there is a short time delay from the small rebound of the puncture member 11 near the second position P2 to the retract of the puncture member 11 along the opposite direction of the first direction X under the drive of the second actuator 31. Along the first direction X, the engagement depth of the shaft base 32 (i.e., the depth of the shaft base 32 being inserted into the slot 410) is defined as H, and the range of motion of the shaft base 32 within the bracket 60 is defined as L, wherein L is greater than H. Since the range of motion of the shaft base 32 within the bracket 60 is greater than the engagement depth of the shaft base 32, the puncture member 11 reaches and stops at the second position P2 after the first locking module 40 unlocks the resetting module 30, thereby ensuring that the first locking module 40 has already unlocked the resetting module 30 when the puncture member 11 reaches the second position P2. In some embodiments, the third actuator 42 is a helical cylindrical spring that is pre-compressed. The third actuator 42 may also be other types of elastic structures suitable to be compressed and provide the driving force to the moving blocks 41.

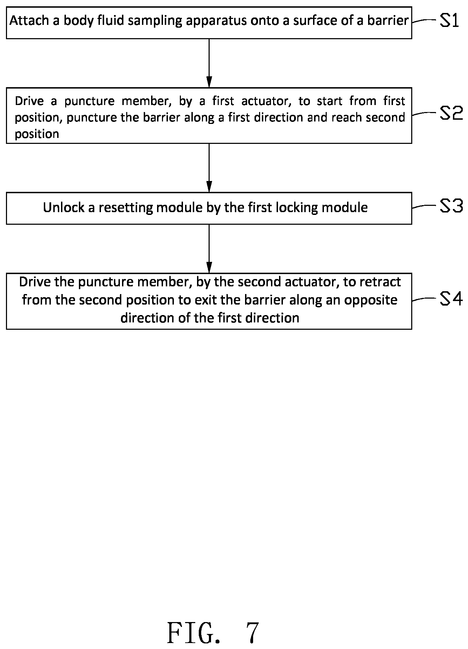

FIG. 7 illustrates a flowchart of a body fluid sampling method according to an embodiment of the present application. According to different needs, the order of steps in the above method may be changed, and some steps may be omitted or merged. The above method includes the following steps.

Step S1, the body fluid sampling apparatus 100 is attached onto a surface of the barrier.

Step S2, the first actuator 21 drives the puncture member 11 to start from the first position P1, puncture the barrier along the first direction X, and then reach the second position P2.

In some embodiments, an external force may be introduced to unlock the puncture module 10 or the driving module 20, thereby causing the first actuator 21 to release the pre-stored driving potential energy that drives the driving shaft 22 to move along the first direction X.

Step S3, the first locking module 40 unlocks the resetting module 30.

Step S4, the second actuator 31 drives the puncture member 11 at the second position P2 to exit the barrier along the opposite direction of the first direction X.

Wherein, the time duration from the puncture member 11 protruding from the sampling port 710 to the puncture member 11 retracting into the housing 70 through the sampling port 710 is defined as T, and the time duration from the puncture member 11 reaching the second position P2 to the puncture member 11 starting to move along the opposite direction under the drive of the second actuator 31 is defined as t, wherein t/T is less than or equal to 10 %.

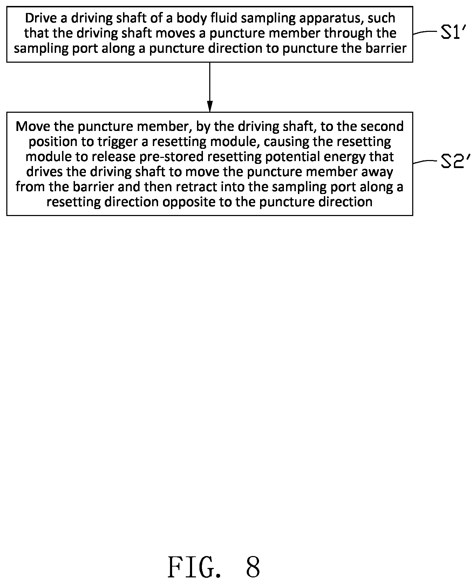

FIG. 8 illustrates a flowchart of a body fluid sampling method according to another embodiment of the present application. According to different needs, the order of steps in the above method may be changed, and some steps may be omitted or merged. The above method includes the following steps.

Step S1', the driving shaft 22 of the body fluid sampling apparatus 100 is driven, such that the driving shaft 22 moves the puncture member 11 through the sampling port 710 along the puncture direction to puncture the barrier. The body fluid sampling apparatus 100 has a bottom surface 71 in contact with the barrier, and a sampling port 710 is opened on the bottom surface 71.

In some embodiments, an external force is introduced to unlock the first actuator 21, thereby causing the first actuator 21 to release the pre-stored driving potential energy that drives the driving shaft 22 to move along the first direction.

Step S2', the driving shaft 22 further moves the puncture member 11 to the second position P2 to trigger the resetting module 30. The resetting module 30 releases the pre-stored resetting potential energy that drives the driving shaft 22 to move the puncture member 11 away from the barrier and then retract into the sampling port 710 along the resetting direction opposite to the puncture direction.

In some embodiments, the driving shaft 22 drives the shaft base 32 to move such that the shaft base 32 releases the first locking module 40, thereby allowing the first locking module 40 to unlock the resetting module 30. Then, the resetting module 30 releases the pre-stored resetting potential energy that drives the shaft base 32 and the driving shaft 22 to move along the resetting direction.

The following will illustrate the present application through specific examples. One skilled in the art would realize that the body fluid sampling method described as follows is an example, and any other suitable devices and methods are within the scope of the present application.

### Example 1

A high-speed camera (model: FASTCAM Mini AX200) was coupled to a microscope, and a side of the body fluid sampling apparatus shown in FIGS. 1 and 2 was placed on the platform of the microscope. A light source was provided above the platform of the microscope, and the direction of the light emitted by the light source was perpendicular to the direction of motion of the puncture member 11. The direction of the microscope objective was also perpendicular to the direction of motion of the puncture member 11.

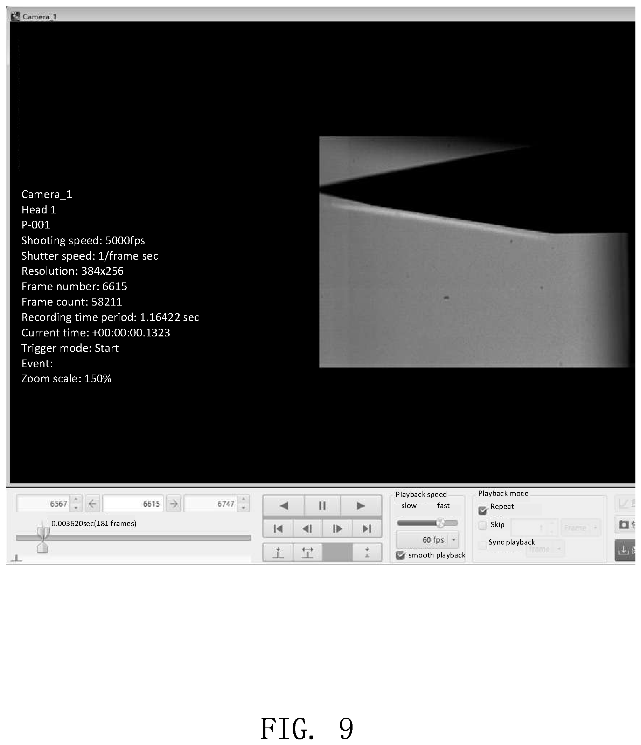

The puncture module 10 was unlocked by an external force, and the first actuator 21 released the driving potential energy for driving the puncture member 11 of the puncture module 10 to move along the first direction X without puncturing any barrier. When the tip of the puncture member 11 passed through the sampling port 710 along the first direction X and reached the second position P2, the first locking module 40 unlockd the resetting module 30, thereby triggering the second actuator 31 to drive the tip of the puncture member 11 to retract from the second position P2 into the housing 70 through the sampling port 710 along the opposite direction of the first direction X. The entire motion process of the puncture member 11 was captured by the high-speed camera, and the capturing parameters were shown in FIG. 9. The entire process from the tip of the puncture member 11 protruding from the sampling port 710 to the tip of the puncture member 11 retracting to the housing 70 was recorded by the 6567^{th} image frame to the 6747^{th} image frame (a total of 181 images). FIG. 9 was the 6615^{th} image frame in which the tip of the puncture member 11 was located near the second position P2.

The time point t0 at which the puncture member 11 starts to move along the first direction X, the time point t1 at which the puncture member 11 protrudes from the sampling port 710, the time point t2 at which the puncture member 11 reaches the second position P2, the time point t3 at which the puncture member 11 starts to retract along the opposite direction of the first direction X under the drive of the second actuator 31, and the time point t4 at which the puncture member 11 retracts into the housing 70 through the sampling port 710 were all recorded. Then, the time duration T from the puncture member 11 protruding from the sampling port 710 to the puncture member 11 retracting to the housing 70 (i.e., the time duration from t1 to t4) was calculated according to the above recorded time points. The time duration t from the puncture member 11 reaching the second position P2 to the puncture member 11 starting to move along the opposite direction under the drive of the second actuator 31 (i.e., the time duration from t2 to t3) was also calculated according to the above recorded time points.

After multiple tests, the time duration T from the puncture member 11 protruding from the sampling port 710 to the puncture member 11 retracting into the housing 70 through the sampling port 710 is always within 5 milliseconds, the time duration t from the puncture member 11 reaching the second position P2 to the puncture member 11 starting to move along the opposite direction under the drive of the second actuator 31 is within 0.5 milliseconds, and t/T is less than or equal to 10 %.

Finally, it should be noted that the above embodiments are used to illustrate the technical solution of the present application and not to limit the present application. Although the present application is described in detail with reference to some exemplary embodiments, those skilled in the art should understand that the technical solution of the present application may be modified or equivalently replaced without departing from the spirit and scope of the technical solution of the present application.

## Claims

1. A body fluid sampling apparatus comprising a bottom surface configured to be in contact with a barrier, **characterized in that**, the body fluid sampling apparatus further comprising:
a puncture module comprising a puncture member;
a driving module comprising a first actuator with a driving potential energy, the first actuator configured to drive the puncture member from a first position to protrude from a sampling port of the bottom surface along a first direction and move to a second position;
a resetting module comprising a second actuator with a resetting potential energy, the second actuator configured to drive the puncture member to retract from the second position into the sampling port along an opposite direction of the first direction; and
a first locking module configured to lock the resetting module, the first locking module is associated with the second position to unlock the resetting module in response to that the puncture member is at the second position, thereby causing the second actuator to release the resetting potential energy which drives the puncture member to retract;
wherein a time duration from the puncture member protruding from the sampling port to the puncture member retracting into the sampling port is defined as T, and T is less than or equal to 5 milliseconds.

2. The body fluid sampling apparatus according to claim 1, **characterized in that**, a time duration from the puncture member reaching the second position to the puncture member starting to move along the opposite direction under drive of the second actuator is defined as t, and t is less than or equal to 0.5 milliseconds.

3. The body fluid sampling apparatus according to claim 1, **characterized in that**, a time duration from the puncture member reaching the second position to the puncture member starting to move along the opposite direction under drive of the second actuator is defined as t, and t/T is less than or equal to 10 %.

4. The body fluid sampling apparatus according to claim 1, **characterized in that**, the driving module further comprises a driving shaft fixed to the puncture module, an outer circumference of the driving shaft comprises a first flange, the first actuator comprises a first elastic member surrounding the driving shaft and elastically resisted against the first locking module and the first flange, and the first locking module is movable relative to the first elastic member and separated from the first elastic member in response to that the puncture member is at the second position.

5. The body fluid sampling apparatus according to claim 4, **characterized in that**, the resetting module further comprises a shaft base linked with the driving shaft, the second actuator comprises a second elastic member surrounding the shaft base, an elastic coefficient of the first elastic member is greater than an elastic coefficient of the second elastic member; the body fluid sampling apparatus further comprises a bracket fixed inside the body fluid sampling apparatus, the shaft base is movable within the bracket along the first direction, and the shaft base is limited to move within the bracket in a predetermined range of motion along the first direction, thereby allowing the puncture member to stop at the second position.

6. The body fluid sampling apparatus according to claim 5, **characterized in that**, the first locking module defines a slot, the first locking module locks the resetting module in a condition that a portion of the shaft base is engaged with the slot; along the first direction, an engagement depth of the shaft base into the slot is defined as H, the predetermined range of motion of the shaft base is defined as L, and L>H.

7. The body fluid sampling apparatus according to claim 1, **characterized in that**, the body fluid sampling apparatus further comprises a second locking module configured to pre-lock the puncture module or pre-lock the driving module, the second locking module is further configured to unlock the puncture module or unlock the driving module under an external force, thereby allowing the first actuator to release the driving potential energy.

8. A body fluid sampling apparatus comprising a bottom surface configured to be in contact with a barrier, **characterized in that**, the body fluid sampling apparatus further comprising:
a puncture module comprising a puncture member;
a driving module comprising a first actuator with a driving potential energy, the first actuator configured to drive the puncture member from a first position to protrude from a sampling port of the bottom surface along a first direction and move to a second position;
a resetting module comprising a second actuator with a resetting potential energy, the second actuator configured to drive the puncture member to retract from the second position into the sampling port along an opposite direction of the first direction; and
a first locking module configured to lock the resetting module, the first locking module associated with the second position to unlock the resetting module in response to that the puncture member is at the second position, thereby causing the second actuator to release the resetting potential energy which drives the puncture member to retract;
wherein a time duration from the puncture member reaching the second position to the puncture member starting to move along the opposite direction under drive of the second actuator is defined as t, and t is less than or equal to 0.5 milliseconds.

9. The body fluid sampling apparatus according to claim 8, **characterized in that**, the driving module further comprises a driving shaft fixed to the puncture module, an outer circumference of the driving shaft comprises a first flange, the first actuator comprises a first elastic member surrounding the driving shaft and elastically resisted against the first locking module and the first flange, and the first locking module is movable relative to the first elastic member and separated from the first elastic member in response to that the puncture member is at the second position.

10. The body fluid sampling apparatus according to claim 9, **characterized in that**, the resetting module further comprises a shaft base linked with the driving shaft, the second actuator comprises a second elastic member surrounding the shaft base, an elastic coefficient of the first elastic member is greater than an elastic coefficient of the second elastic member; the body fluid sampling apparatus further comprises a bracket fixed inside the body fluid sampling apparatus, the shaft base is movable within the bracket along the first direction, and the shaft base is limited to move within the bracket in a predetermined range of motion along the first direction, thereby allowing the puncture member to stop at the second position.

11. The body fluid sampling apparatus according to claim 10, **characterized in that**, the first locking module defines a slot, the first locking module locks the resetting module in a condition that a portion of the shaft base is engaged with the slot; along the first direction, an engagement depth of the shaft base into the slot is defined as H, the predetermined range of motion of the shaft base is defined as L, and L>H.

12. The body fluid sampling apparatus according to claim 8, **characterized in that**, the body fluid sampling apparatus further comprises a second locking module configured to pre-lock the puncture module or pre-lock the driving module, wherein the second locking module is further configured to unlock the puncture module or unlock the driving module under an external force, thereby allowing the first actuator to release the driving potential energy.

13. A body fluid sampling apparatus comprising a bottom surface configured to be in contact with a barrier, **characterized in that**, the body fluid sampling apparatus further comprising:
a puncture module comprising a puncture member;
a driving module comprising a first actuator with a driving potential energy, the first actuator configured to drive the puncture member from a first position to protrude from a sampling port of the bottom surface along a first direction and move to a second position;
a resetting module comprising a second actuator with a resetting potential energy, the second actuator configured to drive the puncture member to retract from the second position into the sampling port along an opposite direction of the first direction; and
a first locking module configured to lock the resetting module, the first locking module associated with the second position to unlock the resetting module in response to that the puncture member is at the second position, thereby causing the second actuator to release the resetting potential energy which drives the puncture member to retract;
wherein a time duration from the puncture member protruding from the sampling port to the puncture member retracting into the sampling port is defined as T, a time duration from the puncture member reaching the second position to the puncture member starting to move along the opposite direction under drive of the second actuator is defined as t, and t/T is less than or equal to 10 %.

14. The body fluid sampling apparatus according to claim 13, **characterized in that**, the driving module further comprises a driving shaft fixed to the puncture module, an outer circumference of the driving shaft comprises a first flange, the first actuator comprises a first elastic member surrounding the driving shaft and elastically resisted against the first locking module and the first flange, and the first locking module is movable relative to the first elastic member and separated from the first elastic member in response to the puncture member reaching the second position.

15. The body fluid sampling apparatus according to claim 14, **characterized in that**, the resetting module further comprises a shaft base linked with the driving shaft, the second actuator comprises a second elastic member surrounding the shaft base, an elastic coefficient of the first elastic member is greater than an elastic coefficient of the second elastic member; the body fluid sampling apparatus further comprises a bracket fixed inside the body fluid sampling apparatus, the shaft base is movable within the bracket along the first direction, and the shaft base is limited to move within the bracket in a predetermined range of motion along the first direction, thereby allowing the puncture member to stop at the second position.

16. The body fluid sampling apparatus according to claim 15, **characterized in that**, the first locking module defines a slot, the first locking module locks the resetting module when a portion of the shaft base is engaged with the slot; along the first direction, an engagement depth of the shaft base into the slot is defined as H, the predetermined range of motion of the shaft base is defined as L, and L>H.

17. The body fluid sampling apparatus according to claim 13, **characterized in that**, the body fluid sampling apparatus further comprises a second locking module configured to pre-lock the puncture module or pre-lock the driving module, the second locking module is further configured to unlock the puncture module or unlock the driving module under an external force, thereby allowing the first actuator to release the driving potential energy.

18. A body fluid sampling method, **characterized by** comprising:
attaching a bottom surface of a body fluid sampling apparatus to a surface of a barrier, wherein the body fluid sampling apparatus comprises a puncture module, a driving module, a resetting module, and a first locking module, the puncture module comprises a puncture member, the driving module comprises a first actuator with a driving potential energy, the resetting module comprises a second actuator with a resetting potential energy, and the first locking module is configured to lock the resetting module;
driving the puncture member, by the first actuator, from a first position to protrude from a sampling port of the bottom surface, puncture the barrier, and move to a second position along a first direction;
unlocking the resetting module by the first locking module; and
driving the puncture member, by the second actuator, to retract from the second position to exit the barrier and into the sampling port along an opposite direction of the first direction;
wherein a time duration from the puncture member protruding from the sampling port to the puncture member retracting to the sampling port is defined as T, a time duration from the puncture member reaching the second position to the puncture member starting to move along the opposite direction under function of the second actuator is defined as t, and t/T is less than or equal to 10 %.

19. The body fluid sampling method according to claim 18, **characterized in that**, driving the puncture member, by the first actuator, from the first position to protrude from the sampling port of the bottom surface along the first direction, comprising:
introducing an external force to unlock the puncture module or the driving module, thereby causing the first actuator to release the driving potential energy which is pre-stored and moves the driving shaft along the first direction.

20. A body fluid sampling method, **characterized by** comprising:
driving a driving shaft of a body fluid sampling apparatus to move, thereby causing the driving shaft to move a puncture member to protrude from a sampling port along a puncture direction to puncture a barrier, wherein the body fluid sampling apparatus comprises a bottom surface in contact with the barrier, and the sampling port is defined at the bottom surface; and
moving the puncture member, by the driving shaft, to a second position to trigger a resetting module, thereby causing the resetting module to release a pre-stored resetting potential energy which drives the driving shaft to move the puncture member away from the barrier and retract into the sampling port along a resetting direction opposite to the puncture direction;
wherein a time duration from the puncture member protruding from the sampling port to the puncture member retracting to the sampling port is defined as T, a time duration from the puncture member reaching the second position to the puncture member starting to move along the opposite direction under drive of the resetting module is defined as t, and t/T is less than or equal to 10 %.

21. The body fluid sampling method according to claim 20, **characterized in that**, driving the driving shaft of the body fluid sampling apparatus to move, thereby causing the driving shaft to move the puncture member to protrude from the sampling port along the puncture direction to puncture the barrier, comprising:
introducing an external force to unlock a first actuator of the body fluid sampling apparatus, thereby causing the first actuator to release a pre-stored driving potential energy which moves the driving shaft along the puncture direction.

22. The body fluid sampling method according to claim 20, **characterized in that**, the sampling apparatus comprises a first locking module associated with the resetting module and a shaft base coupled between the first locking module and the resetting module;
moving the puncture member, by the driving shaft, to the second position to trigger the resetting module, comprising:
moving the shaft base by the driving shaft such that the shaft base releases the first locking module, thereby causing the first locking module to unlock the resetting module; and
releasing the pre-stored resetting potential energy, by the resetting module, to drive the shaft base and the driving shaft to move along the resetting direction.
